# EUROPEAN PATENT SPECIFICATION

(11) **EP 3 302 417 B1**
(45) Date of publication and mention of the grant of the patent: **15.04.2020**
(21) Application number: 16800499.2
(22) Date of filing: 13.05.2016
(51) Int. Cl.: A61K 8/39, A61K 8/891, A61K 8/06, A61Q 5/02

(54) **METHOD FOR FORMING A STYLING HAIR CARE COMPOSITION**
VERFAHREN ZUR HERSTELLUNG EINER ZUSAMMENSETZUNG FÜR HAARSTYLING
PROCÉDÉ POUR LA FORMATION D'UNE COMPOSITION DE SOINS CAPIILLAIRES COIFFANTE

(30) Priority: 26.05.2015 US 201562166340 P
(43) Date of publication of application: 11.04.2018
(73) Proprietor: ELC Management LLC, Melville, NY 11747 (US)
(72) Inventor: SASIK, Camille, Minnetonka, Minnesota 55345 (US); KEEN, Nathan Andrew, Anoka, Minnesota 55303 (US); HAWKINS, Geoffrey, Yardley, Pennsylvania 19067 (US)
(74) Representative: Dehns
(86) International application number: PCT/US2016/032347
(87) International publication number: WO 2016/191130

(56) References cited:
- US-A1- 2012 110 752
- US-A1- 2012 189 665
- US-A1- 2014 308 227
- US-B1- 9 034 305
- US-B2- 8 932 569
- DATABASE GNPD [Online] MINTEL; 17 March 2011 (2011-03-17), anonymous: "Anti-Frizz Leave-In Cream", XP055523184, retrieved from www.gnpd.com Database accession no. 1504823
- DATABASE GNPD [Online] MINTEL; 9 September 2010 (2010-09-09), anonymous: "Shampoo", XP055523188, retrieved from www.gnpd.com Database accession no. 1390873

## Description

### FIELD OF THE INVENTION

The present invention relates to a method for forming personal care compositions comprising a high internal phase emulsion. The styling polymer system is capable of delivering volumizing, fixative, and conditioning benefits as a component of a rinse-off or leave-on personal care composition.

### BACKGROUND OF THE INVENTION

Many shampoo compositions provide acceptable cleaning but provide little or no styling benefits, e.g., body, hold, curl retention, stiffness. To realize such benefits, separate cleaning and styling products are often used.

Recently, hair shampoo compositions have been developed which can provide cleaning and styling performance from a single product. Many of these products contain styling polymers in a compatible shampoo base. To prepare such products, the styling polymer can be dispersed in the surfactant phase of the shampoo composition, and deposited onto the hair to form a thin film on the hair shaft. The polymers provide improved hairstyle benefits such as body, hold, and curl retention. However, current approaches for delivering styling polymers, whether deposited onto the hair via a liquid carrier or by dispersion, frequently leave the hair feeling sticky or tacky.

Based on the foregoing, there is a need for compositions which can deliver hold and soft feel benefits from rinse off formulations, such as a shampoo or conditioner. There is also an ongoing need for naturally-based hair care compositions which confer volume to the hair, improve the styling attributes and enhance the wet combability, and which do not leave the hair stiff or excessively sticky.

### SUMMARY OF THE INVENTION

The present invention relates to a method for forming a styling personal care composition comprising the steps of first combining a polyglyceryl fatty acid ester with water to create a structured external phase premix and second combining the premix with an acrylates/dimethicone copolymer/methyl trimethicone to form a gelled emulsion. The emulsion is suitable for use in cosmetic and personal care formulations to provide benefits including enhanced skin and hair feel. In particular, the compositions are effective for
providing an enhanced styling benefit to hair. Once the gelled emulsion is formed, other optional ingredients may be added to the composition as described herein.

### DETAILED DESCRIPTION OF THE INVENTION

While the specification concludes with claims that particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level, and, therefore, do not include solvents or by-products that may be included in commercially available materials, unless otherwise specified. The term "weight percent" may be denoted as "wt.%" herein.

All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

The term "gelled emulsion", as used herein, means an emulsified system which is thickened, yet easily dispensed from its container.

The term "personal care composition" as used herein shall include personal cleansing and styling compositions, such as for example, hair shampoos, hair conditioners, styling creams and gels, aerosol hair fixatives, and other products for use in personal cleansing and grooming.

The term "polymer" as used herein shall include materials whether made by polymerization of one type of monomer or made by two (*i.e*., copolymers) or more types of monomers.

The term "solid particle" as used herein means a particle that is not a liquid or a gas.

The term "water-soluble" as used herein, means that the polymer is soluble in water in the present composition. In general, the polymer should be soluble at 25°C at a concentration
of at least 0.1% by weight of the water solvent, preferably at least 1%, more preferably at least 5%, most preferably at least 15%.

The term "water-insoluble" as used herein, means that a compound is not soluble in water in the present composition. Thus, the compound is not miscible with water.

### High Internal Phase Emulsion

The personal care compositions formed by the method of the invention comprise a high internal phase emulsion. The emulsion comprises an acrylates/dimethicone copolymer/methyl trimethicone. In order to form a stable emulsion, the acrylates/dimethicone copolymer/methyl trimethicone is stabilized by a mixture of a polyglyceryl fatty acid ester and water.

Polyglyceryl esters (PGE's) are typically formed by esterification of polyglyceryl molecules with fatty acids or by alcoholysis of a vegetable oil with a polyglyceryl. As a class, they have a wide variety of HLB values, which make them suitable for both w/o and o/w systems. They prevent agglomeration in several cosmetic applications including physical sun screen agents. As used herein, the PGE is employed to stabilize the acrylates/dimethicone copolymer/methyl polymer in the high internal phase emulsion.

Suitable PGE's include, for example, Polyglyceryl-8 Caprylate/Caprate, Polyglyceryl-8 Laurate, Polyglyceryl-9 Laurate, Polyglyceryl-10 Laurate, Polyglyceryl-8 Cocoate, Polyglyceryl-9 Cocoate, Polyglyceryl-10 Cocoate, Polyglyceryl-11 Cocoate, Polyglyceryl-12 Cocoate, Polyglyceryl-8 Myristate, Polyglyceryl-9 Myristate, Polyglyceryl-10 Myristate, Polyglyceryl-11 Myristate, Polyglyceryl-12 Myristate, Polyglyceryl-8 Palmitate, Polyglyceryl-9 Palmitate, Polyglyceryl-10 Palmitate, Polyglyceryl-11 Palmitate, Polyglyceryl-12 Palmitate, Polyglyceryl-10 Oleate, Polyglyceryl-11 Oleate, Polyglyceryl-12 Oleate, Polyglyceryl-10 Stearate, Polyglyceryl-12 Stearate, Polyglyceryl-14 Stearate and Polyglyceryl-14 Oleate and combinations thereof. Particularly preferred is Polyglyceryl-10 Laurate.

The acrylates/dimethicone copolymer/methyl trimethicone is a graft copolymer of acrylic polymer and dimethylpolysiloxane dissolved in methyl trimethicone. The volatile content evaporates, leaving a water and oil resistant film. The copolymer (acrylates/dimethicone), which is a film-forming agent, has properties of both an acrylic resin and a silicone. The preferred acrylates/dimethicone copolymer /methyl trimethicone is commercially available from Shin Etsu, under the tradename KP-549P.

To form the high internal phase emulsion, the polyglyceryl fatty acid ester and water are combined, for example by impeller mixing, to form a structured external phase. These components are generally present at a ratio of from about 2:1 to about 1:2. Preferably, the components are present at a ratio of about 1:1.

Once the structured phase is formed, it is slowly combined with the acrylates/dimethicone copolymer/methyl trimethicone, while mixing with a propeller, to form a high internal phase emulsion. The ratio of the acrylates/dimethicone copolymer/methyl trimethicone to the structured external phase may be at least about 80:20, more preferably about 90:10, and most preferably about 95:5.

After formation as a premix, the final high internal phase emulsion is suitable for incorporation into various personal care compositions. In particular, the personal care compositions may be in the form of rinse-off shampoo and conditioner compositions. Such compositions may comprise components discussed hereinafter.

### Oily Conditioning Agent

In one embodiment, the personal care compositions comprise one or more oily conditioning agents. Oily conditioning agents include materials which are used to give a particular conditioning benefit to hair and/or skin. In hair treatment compositions, suitable conditioning agents are those which deliver one or more benefits relating to shine, softness, combability, antistatic properties, wet-handling, damage, manageability, body, and greasiness. The oily conditioning agents useful in the compositions of the present invention typically comprise a water-insoluble, water-dispersible, non-volatile, liquid that forms emulsified, liquid particles. Suitable oily conditioning agents for use in the composition are those conditioning agents characterized generally as silicones (*e.g*., silicone oils, cationic silicones, silicone gums, high refractive silicones, and silicone resins), organic conditioning oils (*e.g*., hydrocarbon oils, polyolefins, and fatty esters) or combinations thereof, or those conditioning agents which otherwise form liquid, dispersed particles in the aqueous surfactant matrix herein.

One or more oily conditioning agents are typically present at a concentration from about 0.01% to about 10%, preferably from about 0.1% to about 8%, more preferably from about 0.2% to about 4%, by weight of the composition.

### Silicone Conditioning Agent

The oily conditioning agents of the personal care compositions are preferably water-insoluble silicone conditioning agents. The silicone conditioning agent may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile
silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions of the present invention preferably have a viscosity, as measured at 25°C, from about 20 to about 2,000,000 [2 × 10⁻⁵ m²/s to 2 m²/s] centistokes ("csk"), more preferably from about 1,000 to about 1,800,000 csk [1 × 10⁻³ m²/s to 1.8 m²/s], even more preferably from about 5,000 to about 1,500,000 csk [5 × 10⁻³ m²/s to 1.5 m²/s], more preferably from about 10,000 to about 1,000,000 csk [10 × 10⁻³ m²/s to 1 m²/s].

Non-volatile silicone oils suitable for use in the personal care compositions may be selected from organo-modified silicones and fluoro-modified silicones. In one embodiment of the present invention, the non-volatile silicone oil is an organo-modified silicone which comprises an organo group selected from the group consisting of alkyl groups, alkenyl groups, hydroxyl groups, amine groups, quaternary groups, carboxyl groups, fatty acid groups, ether groups, ester groups, mercapto groups, sulfate groups, sulfonate groups, phosphate groups, propylene oxide groups, and ethylene oxide groups.

Non-limiting examples of such organo-modified silicones may include those sold by the company OSI under the trade names Silwet L-720®, Silwet L-7002®, Silwet L-7600®, Silwet L-7604®, Silwet L-7605®, Silwet L-7607®, Silwet 1614, Silwet L-7657®, Silwet L-7200®, Silwet L7230®, Silsoft 305®, Silsoft 820®, Silsoft 880®, Tego wet 260®, Tegowet 500®, Tegowet 505 and Tegowet 510®. Most preferred is dimethicone PEG-8 polyacrylate, sold under the trade name Silsoft™ SurFace PF from Momentive.

In a preferred embodiment of the present invention, the non-volatile silicone oil is dimethicone.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

Silicone fluids suitable for use in the personal care compositions are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984). A particularly preferred silicone fluid is Dow Corning 1503 fluid.

### Organic Conditioning Oils

The oily conditioning agent may also comprise at least one organic conditioning oil, either alone or in combination with other conditioning agents, such as the silicones described above.

### Hydrocarbon Oils

Suitable organic conditioning oils for use as conditioning agents in the personal care compositions include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about C₁₂ to about C₁₉. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include 2, 2, 4, 4, 6, 6, 8, 8-dimethyl-10-methylundecane and 2, 2, 4, 4, 6, 6-dimethyl-8-methylnonane, available from Permethyl Corporation. A preferred hydrocarbon polymer is polybutene, such as the copolymer of isobutylene and butene, which is commercially available as L-14 polybutene from Amoco Chemical Corporation.

### Polyolefins

Organic conditioning oils for use in the personal care compositions can also include liquid polyolefins, more preferably liquid poly-α-olefins, more preferably hydrogenated liquid poly-α-olefins. Polyolefins for use herein are prepared by polymerization of C₄ to about C₁₄ olefenic monomers, preferably from about C₆ to about C₁₂.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents.

### Fatty Esters

Other suitable organic conditioning oils for use as the conditioning agent in the personal care compositions include fatty esters having at least 10 carbon atoms. These fatty esters include esters with hydrocarbyl chains derived from fatty acids or alcohols. The hydrocarbyl radicals of the fatty esters hereof may include or have covalently bonded thereto other compatible functionalities, such as amides and alkoxy moieties (*e.g*., ethoxy or ether linkages, *etc*.).

Specific examples of preferred fatty esters include, but are not limited to, isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the personal care compositions are those known as polyhydric alcohol esters. Such polyhydric alcohol esters include alkylene glycol esters.

Still other fatty esters suitable for use in the personal care compositions are glycerides, including, but not limited to, mono-, di-, and tri-glycerides, preferably di- and tri-glycerides, more preferably triglycerides. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

### Fluorinated Conditioning Compounds

Fluorinated compounds suitable for delivering conditioning to hair or skin as organic conditioning oils include perfluoropolyethers, perfluorinated olefins, fluorine based specialty polymers that may be in a fluid or elastomer form similar to the silicone fluids previously described, and perfluorinated dimethicones. Specific non-limiting examples of suitable fluorinated compounds include the Fomblin product line from Ausimont which includes HC/04, HC/25, HC01, HC/02, HC/03; Dioctyldodecyl Fluoroeptyl Citrate, commonly called Biosil Basics Fluoro Gerbet 3.5 supplied by Biosil Technologies; and Biosil Basics Fluorosil LF also supplied by Biosil Technologies.

### Alkyl Glucosides and Alkyl Glucoside Derivatives

Suitable organic conditioning oils for use in the personal care compositions include, but are not limited to, alkyl glucosides and alkyl glucoside derivatives. Specific non-limiting examples of suitable alkyl glucosides and alkyl glucoside derivatives include Glucam E-10, Glucam E-20, Glucam P-10, and Glucquat 125 commercially available from Amerchol.

### Additional Components

The personal care compositions may further comprise one or more additional components known for use in personal care products, provided that the additional components are physically and chemically compatible with the essential components described herein, or do not otherwise unduly impair product stability, aesthetics or performance. Individual concentrations of such additional components may range from about 0.001% to about 10% by weight of the personal care compositions.

Non-limiting examples of additional components for use in the composition include natural cationic deposition polymers, synthetic cationic deposition polymers, anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, chelants, proteins, skin active agents, sunscreens, UV absorbers, and vitamins.

### Cellulose or Guar Cationic Deposition Polymers

The personal care compositions may also include cellulose or guar cationic deposition polymers. Cellulose or glactomannan cationic deposition polymers are preferred. Generally, such cellulose or guar cationic deposition polymers may be present at a concentration from about 0.05% to about 5%, by weight of the composition. Suitable cellulose or guar cationic deposition polymers have a molecular weight of greater than about 5,000. Preferably, the cellulose or guar cationic deposition polymers have a molecular weight of greater than about 200,000. Additionally, such cellulose or guar deposition polymers have a charge density from about 0.15 meq/g to about 4.0 meq/g at the pH of intended use of the personal care composition, which pH will generally range from about pH 3 to about pH 9, preferably between about pH 4 and about pH 8. The pH of the compositions of the present invention are measured neat.

Suitable cellulose or guar cationic polymers include those which conform to the following formula: 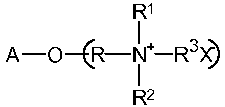 wherein A is an anhydroglucose residual group, such as a cellulose anhydroglucose residual; R is an alkylene oxyalkylene, polyoxyalkylene, or hydroxyalkylene group, or combination thereof; R¹, R², and R³ independently are alkyl, aryl, alkylaryl, arylalkyl, alkoxyalkyl, or alkoxyaryl groups, each group containing up to about 18 carbon atoms, and the total number of carbon atoms for each cationic moiety (*i.e*., the sum of carbon atoms in R¹, R² and R³) preferably being about 20 or less; and X is an anionic counterion. Non-limiting examples of such counterions include halides (*e.g*., chlorine, fluorine, bromine, iodine), sulfate and methylsulfate. The degree of cationic substitution in these polysaccharide polymers is typically from about 0.01 to about 1 cationic groups per anhydroglucose unit.

In one embodiment of the invention, the cellulose or guar cationic polymers are salts of hydroxyethyl cellulose reacted with trimethyl ammonium substituted epoxide, referred to in the industry (CTFA) as Polyquaternium 10 and available from Amerchol Corp. (Edison, N.J., USA).

### Particles

The personal care compositions optionally may comprise additional particles. Preferably, particles useful in the present invention are dispersed water-insoluble particles. Particles useful in the present invention can be inorganic, synthetic, or semisynthetic. In the compositions, it is preferable to incorporate no more than about 20%, more preferably no more than about 10% and even more preferably no more than 2%, by weight of the composition, of particles. In an embodiment of the present invention, the additional particles have an average particle size of less than about 300 µm.

Non-limiting examples of inorganic particles include colloidal silicas, fumed silicas, precipitated silicas, silica gels, magnesium silicate, glass particles, talcs, micas, sericites, and various natural and synthetic clays including bentonites, hectorites, and montmorillonites.

Examples of synthetic particles include silicone resins, poly(meth)acrylates, polyethylene, polyester, polypropylene, polystyrene, polyurethane, polyamide (*e.g*., Nylon®), epoxy resins, urea resins, acrylic powders, and the like.

Non-limiting examples of hybrid particles include sericite & crosslinked polystyrene hybrid powder, and mica and silica hybrid powder.

### Opacifying Agents

The personal care compositions may also contain one or more opacifying agents. Opacifying agents are typically used to impart desired aesthetic benefits to the composition, such as color or pearlescence. In the compositions, it is preferable to incorporate no more than about 20%, more preferably no more than about 10% and even more preferably no more than 2%, by weight of the composition, of opacifying agents.

Suitable opacifying agents include, for example, fumed silica, polymethylmethacrylate, micronized Teflon®, boron nitride, barium sulfate, acrylate polymers, aluminum silicate, aluminum starch octenylsuccinate, calcium silicate, cellulose, chalk, corn starch, diatomaceous earth, Fuller's earth, glyceryl starch, hydrated silica, magnesium carbonate, magnesium hydroxide, magnesium oxide, magnesium trisilicate, maltodextrin, microcrystaline cellulose, rice starch, silica, titanium dioxide, zinc laurate, zinc myristate, zinc neodecanoate, zinc rosinate, zinc stearate, polyethylene, alumina, attapulgite, calcium carbonate, calcium silicate, dextran, nylon, silica silylate, silk powder, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature.

The opacifying agents may also comprise various organic and inorganic pigments. The organic pigments are generally various aromatic types including azo, indigoid, triphenylmethane, anthraquinone, and xanthine dyes. Inorganic pigments include iron oxides, ultramarine and chromium or chromium hydroxide colors, and mixtures thereof.

### Suspending Agents

The personal care compositions may further comprise a suspending agent at concentrations effective for suspending water-insoluble material in dispersed form in the compositions or for modifying the viscosity of the composition. Such concentrations generally range from about 0.1% to about 10%, preferably from about 0.3% to about 5.0%, by weight of the composition, of suspending agent.

Suspending agents useful herein include anionic polymers and nonionic polymers. Useful herein are vinyl polymers such as cross linked acrylic acid polymers with the CTFA name Carbomer.

### Paraffinic Hydrocarbons

The personal care compositions may contain one or more paraffinic hydrocarbons. Paraffinic hydrocarbons suitable for use in compositions include those materials which are known for use in personal care compositions, such as those having a vapor pressure at 1 atm [101 kPa] of equal to or greater than about 21 °C (about 70°F). Non-limiting examples include pentane and isopentane.

### Propellants

The personal care composition also may contain one or more propellants. Propellants suitable for use in compositions include those materials which are known for use in personal care compositions, such as liquefied gas propellants and compressed gas propellants. Suitable propellants have a vapor pressure at 1 atm [101 kPa] of less than about 21 °C (about 70°F). Non-limiting examples of suitable propellants are alkanes, isoalkanes, haloalkanes, dimethyl ether, nitrogen, nitrous oxide, carbon dioxide, and mixtures thereof.

### Other Optional Components

The personal care compositions may contain fragrance.

The personal care compositions may also contain water-soluble and water-insoluble vitamins such as vitamins B1, B2, B6, B12, C, pantothenic acid, pantothenyl ethyl ether, panthenol, biotin and their derivatives, and vitamins A, D, E, and their derivatives. The compositions may also contain water-soluble and water-insoluble amino acids such as asparagine, alanine, indole, glutamic acid and their salts, and tyrosine, tryptamine, lysine, histadine and their salts.

The personal care compositions may contain a mono- or divalent salt such as sodium chloride.

The personal care compositions may also contain chelating agents.

The personal care compositions may further comprise materials useful for hair loss prevention and hair growth stimulants or agents.

### Method of Treating Hair or Skin

The personal care compositions of the may be used in a conventional manner for conditioning, styling, and volumizing hair. A method of treating hair using a composition formed according to the present invention may comprise applying the composition to the hair. More specifically, an effective amount of the personal care composition may be applied to the hair, which has preferably been wetted with water, and preferably shampooed. The composition may be applied to hair via an aerosol or pump-type sprayer in an amount effective to achieve conditioning, volumizing, and hair fixing benefits. Such effective amounts generally range from about 1 g to about 50 g, preferably from about 1 g to about 20 g. Application to the hair typically includes working the composition through the hair such that most or all of the hair is contacted with the composition. It is not necessary to rinse the composition from hair after application as it is preferably used as a leave-in composition.

### EXAMPLES

All parts, percentages, and ratios herein are by weight unless otherwise specified. Some components may come from suppliers as dilute solutions. The levels given reflect the weight percent of the active material, unless otherwise specified.

The compositions illustrated in the following Examples illustrate specific embodiments of the compositions formed according to the present invention, but are not intended to be limiting thereof. Other modifications can be undertaken by the skilled artisan without departing from the scope of this invention as defined in the claims. These exemplified embodiments of the composition formed according to the present invention provide enhanced deposition of conditioning agents to the hair and/or skin.

The compositions illustrated in the following Examples are prepared by conventional formulation and mixing methods, an example of which is described above. All exemplified amounts are listed as weight percents and exclude minor materials such as diluents, preservatives, color solutions, imagery ingredients, botanicals, and so forth, unless otherwise specified.

The high internal phase emulsion is formed by mixing a small amount of the ester (2.5-5%) with water, which creates a structured external phase. Up to about 95% acrylates/dimethicone copolymer/methyl trimethicone is then added to the ester/water premix with stirring, which results in a clear, gelled emulsion.

| **High Internal Phase Emulsion Examples** | **I.** | **II.** | **III.** | **IV.** |
|---|---|---|---|---|
| **WATER\AQUA\EAU** | 2.5 | 5 | 5 | 4 |
| **POLYGLYCERYL-10 LAURATE¹** | 2.5 | 5 | 4 | 3 |
| **ACRYLATES/DIMETHICONE COPOLYMER/METHYL TRIMETHICONE²** | 95 | 95 | 91 | 93 |

| | | | | |
|---|---|---|---|---|
| 1 BARSOLVE NS-100 2 KP-549P | | | | |

The high internal phase emulsions, exemplified above (I.-IV.) may then be incorporated into the various personal care compositions exemplified below. The high internal phase emulsions will be designated as "HIE I.-IV.", respectively, in the examples hereinafter to illustrate incorporation of the high internal phase emulsions illustrated in the examples above.

The following are representative of shampoo compositions:

| **Shampoo Composition** | **1** | **2** | **3** | **4** | **5** | **6** | **7** | **8** |
|---|---|---|---|---|---|---|---|---|
| **WATER\AQUA\EAU** | q.s. | q.s. | q.s. | q.s. | q.s. | q.s. | q.s. | q.s. |
| **Sodium Chloride** | 3.00 | 3.00 | 3.00 | 3.00 | 3.00 | 3.00 | 3.00 | 3.00 |
| **Sodium Gluconate** | 0.10 | 0.10 | 0.10 | 0.10 | 0.10 | 0.10 | 0.10 | 0.10 |
| **Cationic Polymer³** | 0.25-1.50 | 0.25-1.50 | 0.25-1.50 | 0.25-1.50 | 0.25-1.50 | 0.25-1.50 | 0.25-1.50 | 0.25-1.50 |
| **1,3-Propanediol** | 2.00-4.00 | 2.00-4.00 | 2.00-4.00 | 2.00-4.00 | 2.00-4.00 | 2.00-4.00 | 2.00-4.00 | 2.00-4.00 |
| **Sodium Methyl Cocyl Taurate** | 7.50 | 7.50 | 7.50 | 7.50 | 7.50 | 7.50 | 7.50 | 7.50 |
| **Sodium lauroyl Methyl Isethionate/Lauric Acid/Sodium Sulfate/Sodium Laurate⁴** | 10.00 | 10.00 | 10.00 | 10.00 | 10.00 | 10.00 | 10.00 | 10.00 |
| **Cocamide MIPA** | 2.50 | 2.50 | 2.50 | 2.50 | 2.50 | 2.50 | 2.50 | 2.50 |
| **Babassuamidiopropyl Betaine** | 9.00 | 9.00 | 9.00 | 9.00 | 9.00 | 9.00 | 9.00 | 9.00 |
| **Glycol Distearate** | 2.00 | 2.00 | 2.00 | 2.00 | 2.00 | 2.00 | 2.00 | 2.00 |
| **Potassium Sorbate** | 0.10 | 0.10 | 0.10 | 0.10 | 0.10 | 0.10 | 0.10 | 0.10 |
| **Methyl Soyate** | 3.00 | 3.00 | 3.00 | 3.00 | 3.00 | 3.00 | 3.00 | 3.00 |
| **Glyceryl Caprylate** | 1.00 | 1.00 | 1.00 | 1.00 | 1.00 | 1.00 | 1.00 | 1.00 |
| **Phenoxyethanol** | 0.50 | 0.50 | 0.50 | 0.50 | 0.50 | 0.50 | 0.50 | 0.50 |
| **DIMETHICONE PEG-8 POLYACRYLATE⁵** | 0.50 | 0.50 | 0.50 | 0.50 | 0.50 | 0.50 | 0.50 | 0.50 |
| **Citric Acid** | 0.50 | 0.50 | 0.50 | 0.50 | 0.50 | 0.50 | 0.50 | 0.50 |
| **HIE I.** | 1.00 | 5.00 | - | - | - | - | - | - |
| **HIE II.** | - | - | 2.00 | 5.00 | - | - | - | - |
| **HIE III.** | - | - | - | - | 3.00 | 5.00 | - | - |
| **HIE IV.** | - | - | - | - | - | - | 1.00 | 4.00 |

| | | | | | | | | |
|---|---|---|---|---|---|---|---|---|
| 3 Jaguar C-17 Guar Hydroxypropyl trimonium chloride from Rhodia 4 ISELUX® from Innospec 5 SILSOFT™ SURFACE PF from Momentive | | | | | | | | |

The following are representative of conditioner compositions:

| **Conditioner Examples** | **9** | **10** | **11** | **12** | **13** | **14** | **15** | **16** |
|---|---|---|---|---|---|---|---|---|
| **WATER\AQUA\EAU** | q.s. | q.s. | q.s. | q.s. | q.s. | q.s. | q.s. | q.s. |
| **STEARALKONIUM CHLORIDE** | 1.50 | 1.50 | 1.50 | 1.50 | 1.50 | 1.50 | 1.50 | 1.50 |
| **CETYL ALCOHOL** | 4.00 | 4.00 | 4.00 | 4.00 | 4.00 | 4.00 | 4.00 | 4.00 |
| **GLYCERYL STEARATE** | 1.80 | 1.80 | 1.80 | 1.80 | 1.80 | 1.80 | 1.80 | 1.80 |
| **GLYCERYL STEARATE/PEG-100 STEARATE** | 1.00 | 1.00 | 1.00 | 1.00 | 1.00 | 1.00 | 1.00 | 1.00 |
| **DISTEARYLDIMONIUM CHLORIDE** | 0.70 | 0.70 | 0.70 | 0.70 | 0.70 | 0.70 | 0.70 | 0.70 |
| **POLYQUATERNIUM-4** | 0.50 | 0.50 | 0.50 | 0.50 | 0.50 | 0.50 | 0.50 | 0.50 |
| **CETRIMONIUM CHLORIDE** | 2.00 | 2.00 | 2.00 | 2.00 | 2.00 | 2.00 | 2.00 | 2.00 |
| **DIMETHICONE PEG-8 POLYACRYLATE** | 0.25 - 3.00 | 0.25 - 3.00 | 0.25 - 3.00 | 0.25 - 3.00 | 0.25 - 3.00 | 0.25 - 3.00 | 0.25 - 3.00 | 0.25 - 3.00 |
| **HIE I.** | 1.00 | 5.00 | | | | | | |
| **HIE II**. | | | 8.00 | 10.00 | | | | |
| **HIE III.** | | | | | 3.00 | 14.00 | | |
| **HIE IV**. | | | | | | | 4.00 | 12.00 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention as defined in the claims. It is therefore intended to cover in the appended claims all such changes and modifications that are within the scope of this invention.

## Claims

1. A method for forming a high internal phase emulsion comprising the steps of:
a) first, combining a polyglyceryl fatty acid ester with water to create a structured external phase premix; and
b) second, combining said premix with an acrylates/dimethicone copolymer/methyl trimethicone to form a gelled emulsion.

2. A method according to claim 1, wherein the ratio of said acrylates/dimethicone copolymer/methyl trimethicone to said structured external phase is at least about 90:10.

3. A method according to claim 2, wherein the ratio of said acrylates/dimethicone copolymer/methyl trimethicone to said structured external phase is at least about 95:5.

4. A method according to claim 1, wherein the ratio of said polyglyceryl fatty acid ester to said water is about 1:1.

5. A method according to claim 1, wherein said high internal phase emulsion is a component of a personal care composition, wherein said personal care composition further comprises a surfactant and a conditioning agent.

6. A method according to claim 5, wherein said conditioning agent is selected from the group consisting of silicone oils, cationic silicones, silicone gums, high refractive silicones, organo-modified silicones, fluoro-modified silicones, silicone resins, hydrocarbon oils, polyolefins, fatty esters, and mixtures thereof.

7. A method according to claim 5, wherein said surfactant is selected from the group consisting of anionic surfactants, cationic surfactants, and amphoteric surfactants.

8. A method according to claim 5, wherein said personal care composition further comprises a cationic deposition polymer.

9. A method according to claim 8, wherein said cationic deposition polymer is selected from the group consisting of cellulose and guar cationic deposition polymers.

10. A method according to claim 5, wherein said personal care composition further comprises particles having an average particle size of less than about 300 µm.

11. A method according to claim 10, wherein said particles are selected from the group consisting of colloidal silicas, fumed silicas, precipitated silicas, silica gels, magnesium silicate, glass particles, talcs, micas, sericites, and clays.

12. A method according to claim 5, wherein said personal care composition further comprises one or more additional ingredients selected from the group consisting of synthetic cationic deposition polymers, anti-dandruff agents, suspending agents, paraffinic hydrocarbons, propellants, viscosity modifiers, dyes, non-volatile solvents, diluents, pearlescent aids, foam boosters, additional surfactants or nonionic cosurfactants, pediculocides, pH adjusting agents, perfumes, preservatives, chelants, proteins, skin active agents, sunscreens, UV absorbers, and vitamins.

13. A method according to claim 5, wherein said high internal phase is present at a level of from about 1% to about 20% by weight of said personal care composition.

## Patentansprüche

1. Verfahren zum Ausbilden einer Hohe-Innere-Phase-Emulsion, umfassend die Schritte:
a) erstens, Kombinieren eines Polyglyceryl-Fettsäureesters mit Wasser zum Erzeugen einer strukturierten Äußere-Phase-Vormischung; und
b) zweitens, Kombinieren der Vormischung mit einem Acrylaten/Dimethikoncopolymer/Methyltrimeticon zum Ausbilden einer gelierten Emulsion.

2. Verfahren nach Anspruch 1, wobei das Verhältnis des Acrylaten/Dimethikoncopolymer/Methyltrimeticons zur strukturierten äußeren Phase mindestens ungefähr 90:10 ist.

3. Verfahren nach Anspruch 2, wobei das Verhältnis des Acrylaten/Dimethikoncopolymer/Methyltrimeticons zur strukturierten äußeren Phase mindestens ungefähr 95:5 ist.

4. Verfahren nach Anspruch 1, wobei das Verhältnis des Polyglyceryl-Fettsäureesters zum Wasser ungefähr 1:1 ist.

5. Verfahren nach Anspruch 1, wobei die Hohe-Innere-Phase-Emulsion eine Komponente einer Körperpflegezusammensetzung ist, wobei die Körperpflegezusammensetzung weiter einen oberflächenaktiven Stoff und ein Konditionierungsmittel umfasst.

6. Verfahren nach Anspruch 5, wobei das Konditionierungsmittel ausgewählt ist aus der Gruppe, die besteht aus Silikonölen, kationischen Silikonen, Silikongummis, hochrefraktiven Silikonen, organomodifizierten Silikonen, fluormodifizierten Silikonen, Silikonharzen, Kohlenwasserstoffölen, Polyolefinen, Fettsäureestern und Mischungen davon.

7. Verfahren nach Anspruch 5, wobei der oberflächenaktive Stoff aus der Gruppe ausgewählt ist, die besteht aus anionischen oberflächenaktiven Stoffen, kationischen oberflächenaktiven Stoffen und amphoteren oberflächenaktiven Stoffen.

8. Verfahren nach Anspruch 5, wobei die Körperpflegezusammensetzung weiter ein kationisches Depositionspolymer umfasst.

9. Verfahren nach Anspruch 8, wobei das kationische Depositionspolymer aus der Gruppe ausgewählt ist, die besteht aus Zellulose und Guar-kationischen Depositionspolymeren.

10. Verfahren nach Anspruch 5, wobei die Körperpflegezusammensetzung weiter Partikel umfasst, die eine durchschnittliche Partikelgröße von weniger als ungefähr 300 pm haben.

11. Verfahren nach Anspruch 10, wobei die Partikel aus der Gruppe ausgewählt sind, die besteht aus Kolloidsiliciumdioxiden, pyrogenen Siliciumdioxiden, gefällten Siliciumdioxiden, Siliciumdioxid-Gelen, Magnesiumsilikat, Glaspartikeln, Talken, Glimmern, Sericiten und Tonen.

12. Verfahren nach Anspruch 5, wobei die Körperpflegezusammensetzung weiter eine oder mehrere Zutaten umfasst, die ausgewählt sind aus der Gruppe, die besteht aus synthetischen kationischen Depositionspolymeren, Anti-Schuppen-Mitteln, Suspensionsmitteln, paraffinischen Kohlenwasserstoffen, Treibmitteln, Viskositätsmodifizieren, Farbstoffen, nichtflüchtigen Lösungsmitteln, Verdünnungsmitteln, Perlglanzhilfen, Schaumverstärkern, zusätzlichen oberflächenaktiven Stoffen oder nichtionischen Kotensiden, Entlausungsmitteln, pH-Einstellmitteln, Parfümen, Konservierungsstoffen, Chelatbildnern, Proteinen, hautaktiven Mitteln, Sonnenschutzmitteln, UV-Absorbern und Vitaminen.

13. Verfahren nach Anspruch 5, wobei die hohe innere Phase bei einem Niveau von ungefähr 1 Gew.-% bis ungefähr 20 Gew.-% der Körperpflegezusammensetzung vorhanden ist.

## Revendications

1. Procédé pour former une émulsion à phase interne élevée, comprenant les étapes consistant :
a) en premier lieu, à combiner un ester d'acide gras de polyglycéryle avec de l'eau pour créer un prémélange de phase externe structurée ; et
b) en second lieu, à combiner le prémélange avec un copolymère acrylates/diméthicone/triméthicone de méthyle pour former une émulsion gélifiée.

2. Procédé selon la revendication 1, dans lequel le rapport entre ledit copolymère acrylates/diméthicone/triméthicone de méthyle et ladite phase externe structurée est d'au moins environ 90:10.

3. Procédé selon la revendication 2, dans lequel le rapport entre ledit copolymère acrylates/diméthicone/triméthicone de méthyle et ladite phase externe structurée est d'au moins environ 95:5.

4. Procédé selon la revendication 1, dans lequel le rapport entre ledit ester d'acide gras de polyglycéryle et ladite eau est d'environ 1:1.

5. Procédé selon la revendication 1, dans lequel ladite émulsion à phase interne élevée est un composant d'une composition de soins personnels, dans lequel ladite composition de soins personnels comprend en outre un tensioactif et un agent de conditionnement.

6. Procédé selon la revendication 5, dans lequel ledit agent de conditionnement est sélectionné dans le groupe constitué par des huiles de silicone, des silicones cationiques, des gommes de silicone, des silicones à indice de réfraction élevé, des silicones organomodifiées, des silicones fluoromodifiées, des résines de silicone, des huiles hydrocarbonées, des polyoléfines, des esters gras et des mélanges de ceux-ci.

7. Procédé selon la revendication 5, dans lequel ledit tensioactif est sélectionné dans le groupe constitué par des tensioactifs anioniques, des tensioactifs cationiques et des tensioactifs amphotériques.

8. Procédé selon la revendication 5, dans lequel ladite composition de soins personnels comprend en outre un polymère de dépôt cationique.

9. Procédé selon la revendication 8, dans lequel ledit polymère de dépôt cationique est sélectionné dans le groupe constitué par des polymères de dépôt cationique à base de cellulose et de guar.

10. Procédé selon la revendication 5, dans lequel ladite composition de soins personnels comprend en outre des particules ayant une taille moyenne des particules inférieure à 300 µm.

11. Procédé selon la revendication 10, dans lequel lesdites particules sont sélectionnées dans le groupe constitué par des silices colloïdales, des silices sublimées, des silices précipitées, des gels de silice, du silicate de magnésium, des particules de verre, des talcs, des micas, des séricites et des argiles.

12. Procédé selon la revendication 5, dans lequel ladite composition de soins personnels comprend en outre un ou plusieurs ingrédients supplémentaires sélectionnés dans le groupe constitué par des polymères de dépôt cationiques synthétiques, des agents antipelliculaires, des agents de suspension, des hydrocarbures paraffiniques, des ergols, des agents modifiant la viscosité, des colorants, des solvants non volatils, des diluants, des aides à un effet nacré, des renforçateurs de mousse, des tensioactifs supplémentaires ou des cotensioactifs non ioniques, des pédiculocides, des agents ajusteurs de pH, des parfums, des conservateurs, des agents chélateurs, des protéines, des agents actifs pour la peau, des écrans solaires, des absorbeurs d'UV et des vitamines.

13. Procédé selon la revendication 5, dans lequel ladite phase interne élevée est présente à un niveau allant d'environ 1 % à environ 20 % en poids de ladite composition de soins personnels.
